# EUROPEAN PATENT SPECIFICATION

(11) **EP 3 377 044 B1**
(45) Date of publication and mention of the grant of the patent: **29.09.2021**
(21) Application number: 16797954.1
(22) Date of filing: 18.11.2016
(51) Int. Cl.: A61K 9/14, A61K 9/16, A61K 9/00, A61K 31/192

(54) **IBUPROFEN COMPOSITIONS FOR DIRECT ORAL ADMINISTRATION**
IBUPROFEN-ZUSAMMENSETZUNGEN ZUR DIREKTEN ORALEN VERABREICHUNG
COMPOSITIONS D'IBUPROFÈNE POUR ADMINISTRATION DIRECTE PAR VOIE ORALE

(30) Priority: 18.11.2015 EP 15195257
(43) Date of publication of application: 26.09.2018
(73) Proprietor: HERMES PHARMA GmbH, 82049 Pullach (DE)
(72) Inventor: HAALA, Josef, 82049 Pullach (DE); ARMSTRONG, Annemarie, 82049 Pullach (DE); GARSUCH, Verena, 82049 Pullach (DE)
(74) Representative: Pharma Patents International AG
(86) International application number: PCT/EP2016/078204
(87) International publication number: WO 2017/085295

(56) References cited:
- EP-A1- 0 582 380
- WO-A1-2013/004123
- WO-A2-2008/091957
- US-A1- 2002 122 823
- DATABASE WPI Week 200748 2007 Thomson Scientific, London, GB; AN 2007-489569 XP002755287, & JP 2007 131561 A (SS PHARM CO) 31 May 2007 (2007-05-31)

## Description

### FIELD

The present invention relates to compositions for the direct oral delivery of ibuprofen without water. The compositions are employed in the treatment of pain, inflammation and/or fever due to the analgesic, anti-inflammatory and/or antipyretic properties of the drug ibuprofen.

### BACKGROUND

In the field of analgesic drugs it is commonly desirable that drug products may be administered to an individual in need thereof as quickly as possible after onset of pain, in order to achieve quickest possible relief. This has brought about compositions for direct oral administration; i.e. compositions which can be orally administered and swallowed easily without the need for additional water. This means that either the compositions contain water and/or another ingestible liquid already (such as aqueous or oily solutions or suspensions), or the formulations are initially dry but are wetted and dissolved or dispersed easily in saliva once ingested orally. Dry formulations are commonly preferred by adults, due to their lower weight/bulk and lower risk of spillage.

However, with direct oral compositions - and in in particular with the dry formulations - the components get in direct contact with the user's, or consumer's tongue, which is often problematic for drugs (or active agents, or pharmaceutically active ingredients (API)) and excipients with poor organoleptic properties (taste, smell, sensations such as burning/stinging/scratching etc.).

Ibuprofen (chemical name: (±)-2-(p-Isobutylphenyl)propionic acid), which is used as an analgesic or anti-inflammatory active ingredient and classified as a so-called nonsteroidal anti-inflammatory drug (NSAID), is one of numerous examples for such drugs, known for its bitter taste and other unpleasant organoleptic sensations, such as scratching, stinging or burning on the mucous membranes in the mouth, tongue and throat region. Various taste-masking attempts for ibuprofen have been described in the literature.

For instance, US 4,788,220 A describes taste neutral pediatric ibuprofen suspensions containing about 1-2 % (w/V) micronized ibuprofen, 1.25-1.5 % (w/V) of a suspension stabilizing combination which consists essentially of xanthan gum, micro crystalline cellulose (MCC), sodium carboxymethylcellulose (Na-CMC) and polysorbate 80; as well as 55-75 % (w/V) of a taste masking combination which consists essentially of sucrose and sorbitol solution. The drawback of this type of formulation is the very low ibuprofen loading which is suitable only for the smaller, pediatric single doses; and the taste is not truly masked but rather 'covered' by the large amounts of sweet-tasting excipients.

Another very common approach, if not the most common, is to apply taste masking coatings to solid formulations and/or the drug as such. For instance, US 5,489,436 A describes chewable tablet of ibuprofen, in which the drug is covered with a taste-masking coating comprising a mixture of dimethylaminoethyl methacrylate and neutral methacrylic acid ester and a cellulose ester such as cellulose acetate, prior to incorporating the coated drug into chewable tablets.

Furthermore, WO 2013/004123 A1 discloses taste-masked ibuprofen compositions in the form of directly compressed, chewable tablets. The drawback of this taste-masking approach is that coating procedures can be complex and time-consuming and that taste-masking relies on the uniformity and integrity of the applied coat.

It is thus an object of the present invention to provide an improved direct oral composition that is easy to prepare, easy to administer in its dry form and has pleasant organoleptic properties, thereby increasing consumer compliance.

Further objects will become apparent on the basis of the following description including the examples, and the patent claims.

### SUMMARY OF THE INVENTION

In a first aspect, the invention provides a pharmaceutical composition for direct oral administration without water formulated as a flowable powder comprising uncoated ibuprofen particles, a hydrophilic, water-soluble and gelling polymer, which is not crosslinked, and at least one further pharmaceutically acceptable excipient, wherein
(a) the mean particle size of the ibuprofen particles ranges from about 200 µm to about 400 µm; and wherein
(b) not more than 10 wt.-% of the ibuprofen particles have a sieve diameter of less than 150 µm, and/or
(c) not more than 5 wt.-% of the ibuprofen particles have a sieve diameter of less than 100 µm

In one embodiment, not more than 7 wt.-% of the ibuprofen particles have a sieve diameter of less than 150 µm, and/or not more than 3 wt.-% of the ibuprofen particles have a sieve diameter of less than 100 µm.

The inventors surprisingly found that the use of ibuprofen particles having a particle size distribution which complies with these limitations and which, accordingly, have a relatively small fine powder fraction, obviates the requirement for coating and/or granulating the ibuprofen particles with taste-masking excipients in order to mask their poor taste in direct oral compositions, and thus reduces the cost and complexity of the production process. As used herein, direct oral compositions are compositions for oral administration without water.

In one specific embodiment of the invention, the pharmaceutical composition is substantially free of an unpleasant, bitter taste and/or another unpleasant organoleptic sensation for at least 20 seconds after being placed into the mouth of a human subject, preferably for at least 30 seconds. This is long enough to swallow a single dose of the composition without experiencing any noteworthy unpleasant organoleptic sensations such as bitter taste and/or scratching, stinging or burning. Substantially free of unpleasant taste(s) and/or other unpleasant organoleptic sensation(s) should be understood in such a way that at least 90 % of users/consumers would rank their perception(s) upon oral ingestion of the composition according to the invention as "very good" (no noticeable unpleasant sensation for 45 s or more), "good" (unpleasant sensation first noticeable after 30 s or more) or "acceptable" (unpleasant sensation first noticeable within 30 s but only to a mild degree for at least 45 s).

In a further embodiment, the pharmaceutical composition comprises ibuprofen particles of which not more than 10 wt.-% have a sieve diameter of 500 µm or more. This is preferred in that it helps to reduce or avoid grittiness and/or foreign body sensations of the composition when placed in the mouth / on the tongue.

The ibuprofen in the pharmaceutical compositions may be provided in a granulated form. An example of a commercial grade of granulated ibuprofen is ibuprofen DC 100, which consists of ibuprofen and is prepared by spray granulation without the use of added excipients. The product is commercially available from Glatt GmbH (Binzen, Germany; distributed via Pharmatrans-Sanaq AG, Basel, Switzerland).

Alternatively - or in addition to the granulation - the ibuprofen particles having the particle size distribution as described above may also be obtained by sieving, for example to remove some or all of the ibuprofen particles with a sieve diameter of 150 µm or less, or of 100 µm or less.

The pharmaceutical composition may, for example, comprise from about 10 wt.-% to about 50 wt.-% ibuprofen particles.

The pharmaceutical composition of the invention further comprises one or more hydrophilic, water-soluble and gelling polymers (herein shortly referred to as the hydrophilic polymer), with the purpose of improving the texture, or consistency, of the composition upon ingestion, e.g. by the swelling and/or gelling and/or thickening of the hydrophilic polymer when in contact with aqueous media such as saliva. The hydrophilic polymer is not crosslinked. In one embodiment, the hydrophilic polymer may be selected from pregelatinised starch, xanthan, carmellose-sodium or combinations thereof.

The weight ratio of the ibuprofen particles to the hydrophilic polymer in the compositions preferably ranges from about 1 : 1 to about 20 : 1, or from about 1 : 1 to about 10 : 1; e.g. about 2 : 1, 4 : 1, 6 : 1, 8 : 1, 12 : 1, 14 : 1 or 16 : 1.

The pharmaceutical composition of the invention comprises at least one further pharmaceutically acceptable excipient in addition to the uncoated ibuprofen particles and the hydrophilic polymer. In one embodiment, said excipient is selected from the group of water-soluble sugars, sugar alcohols and oligosaccharides; or from the group of flavourings, cooling agents, salivation stimulating excipients, acidity regulators, lubricants and flow-regulating agents. Optionally, the further excipient may also be provided in the form of granules; e.g. granules having a particle size distribution similar to that of the ibuprofen particles. In a specific embodiment, the sugar or sugar alcohol may be selected from the group of sucrose, maltitol, mannitol, sorbitol, xylitol, and mixtures thereof.

In one embodiment, a primary package unit comprising a single dose of the pharmaceutical composition is provided, wherein the package unit is selected from stick packs, sachets, ampoules, or vials. Further, the primary package unit may comprise about 50 mg to 800 mg ibuprofen.

In a specific aspect of the invention, the pharmaceutical composition comprises spray-granulated ibuprofen particles, at least one hydrophilic polymer selected from pregelatinised starch, xanthan, carmellose-sodium and combinations thereof; and at least one further pharmaceutically acceptable excipient including
a) a blend of water-soluble sugars, sugar alcohols and/or oligosaccharides comprising at least two components selected from sorbitol, xylitol, mannitol, sucrose and maltitol;
b) a salivation stimulating excipient selected from citric acid, monosodium citrate disodium citrate and combinations thereof;
c) one or more flavourings selected from flavours and sugar substituents; and
d) a lubricant and/or flow-regulating agent.

In a yet further aspect, the invention provides a process for the preparation of a pharmaceutical composition as described above, comprising the steps of:
(i) providing uncoated ibuprofen particles
   (a) which exhibit a mean particle size of ranging from about 200 µm to about 400 µm; and
   (b) of which not more than 10 wt.-% have a sieve diameter of less than 150 µm, and/or
   (c) of which not more than 5 wt.-% have a sieve diameter of less than 100 µm;
(ii) providing a hydrophilic polymer;
(iii) providing at least one further pharmaceutically acceptable excipient; and
(iv) blending all components provided in steps (i) to (iii) to form a homogenous powder composition.

Optionally, step (i) in this process includes a granulation step and/or a sieving step to obtain the ibuprofen particles of the particle sizes described.

The compositions of the invention may be used for the treatment of pain, inflammation and/or fever due to the analgesic, anti-inflammatory and/or antipyretic properties of the drug ibuprofen.

### DETAILED DESCRIPTION OF THE INVENTION

In a first aspect, the invention provides a pharmaceutical composition for direct oral administration without water formulated as a flowable powder comprising uncoated ibuprofen particles, a hydrophilic, water-soluble and gelling polymer, which is not crosslinked and at least one further pharmaceutically acceptable excipient, wherein
(a) the mean particle size of the ibuprofen particles ranges from about 200 µm to about 400 µm; and wherein
(b) not more than 10 wt.-% of the ibuprofen particles have a sieve diameter of less than 150 µm, and/or
(c) not more than 5 wt.-% of the ibuprofen particles have a sieve diameter of less than 100 µm.

Unless specified otherwise, the term 'ibuprofen particles' as used herein refers to the uncoated particles; i.e. there is no excipient layer covering, or adhering to, the surface of these particles which would prevent or limit wetting of the particles or delay their dissolution when in contact with aqueous media such as saliva. Furthermore, the uncoated ibuprofen particles preferably exhibit high drug content, such as at least 90 wt.-%, or at least 93 wt.-%, or at least 95 wt.-%, or at least 96 wt.-%, or at least 97 wt.-%, or at least 98 wt.-%, or at least 99 wt.-% or 100 wt.-%; and do not contain excipients dedicated to taste-masking such as poorly water-soluble polymers or the like.

Preferably, the ibuprofen as used herein is provided in the form of its free acid, either in racemic form or in the form of its enantiomers, such as the S-enantiomer (called dexibuprofen); this applies for all ibuprofen particle sizes described herein and irrespective of the nature and/or amount of further components in the pharmaceutical compositions.

Furthermore, for reasons of simplicity and brevity the hydrophilic, water-soluble, gelling polymer will be referred to herein as the 'hydrophilic polymer'. Again, this is valid for all ibuprofen particle sizes described herein and irrespective of the nature and/or amount of the other components in the pharmaceutical compositions.

Any solubility provisions used herein shall be understood as solubilities in water and follow pharmacopoeial standards (e.g. European Pharmacopeia; Ph.Eur.) unless where specified otherwise. The common expression 'poorly water-soluble' refers to substances, for instance polymers, which are defined by the Ph.Eur. as sparingly soluble or slightly soluble, in other words substances requiring between 30 mL and 1000 mL solvent per gram of solute. The common term 'water-insoluble' as used herein refers to substances which are very slightly soluble or practically insoluble, in other words substances requiring >1000 mL per gram of solute.

The mean particle size as used herein is a weighted arithmetic mean value as measured by dynamic image analysis, such as according to ISO 13322-2. Unless mentioned otherwise, the particle size values provided herein (either measured or calculated/derived from measured values) were determined using a Camsizer® XT device (Retsch Technology GmbH, Haan, Germany) equipped with the X-Jet plug-in cartridge and its related software.

The Camsizer® set-up employs a dynamic imaging technique, rather than actual 'physical' sieving of the particles. Samples are dispersed by pressurised air and passed through a gap illuminated by two bright, pulsed LED light sources. The images of the dispersed particles (more specifically of their shadows, or projections) are then recorded by two digital cameras and analysed for size and shape in order to determine a variety of length and width descriptors for the particles, as required e.g. by ISO norm 13322-2 (on particle size analysis via dynamic imaging); e.g. the width of the particle, i.e. the shortest chord of the measured set of maximum chords of a particle's projection (Camsizer parameter X_{c min}, also called the minimum largest chord diameter); or the maximum Feret diameter (Camsizer parameter X_{Fe max}); or the aspect ratio AR (Camsizer parameter b/l₃).

The particle width is the preferred particle size parameter in the present invention since this parameter is most closely related to physical screening using sieving manoeuvres. A particle with a width smaller than a sieve aperture is able to pass the sieve even if the length of such particle is larger than the width. Thus the terms 'particle size' and 'sieve diameter' are nearly the same in the context of the invention and may be used interchangeably herein.

Using width values, mass fractions within specific particle size ranges or cumulative mass fractions of all particles smaller than a specific particle size x (i.e. the fraction which would pass the 'virtual sieve' of diameter x) may be derived, and further the weighted arithmetic mean particle size as well as D10, D50 and D90 (the particle size which is passed by a mass fraction of 10 %, 50 % and 90 % of the particles, respectively); similar to usual sieve analysis, however with a far higher precision.

The Camsizer® XT device is preferred for particle size determinations according to the invention since it allows for the precise and reproducible analysis of particle sizes of fine powders down to 1 micrometre; a precision not achievable with conventional sieve analysis on sieving towers. This, however, should not be misinterpreted in such a way as to exclude, or prohibit, the use of conventional sieves or sieving towers for preliminary sieve diameter determinations and/or for the classification of particles; e.g. in order to remove ibuprofen particles with a low particle size, such as of 150 µm or less, or 100 µm or less. Furthermore, this does not exclude, or prohibit, the use of laser diffraction or any other established methods for preliminary particle size determinations. While in such cases the particle sizes determined may differ from those obtained with the Camsizer® set-up, the skilled person will appreciate, that preliminary evaluation of the particle size distribution is nonetheless possible in most cases; in case of doubt, the results obtained with a Camsizer® prevail.

In another specific embodiment, not more than 7 wt.-% of the ibuprofen particles have a sieve diameter of less than 150 µm, and/or not more than 3 wt.-% of the ibuprofen particles have a sieve diameter of less than 100 µm. In a further embodiment, not more than 2 wt.-% of the ibuprofen particles have a sieve diameter of less than 50 µm.

The inventors have surprisingly found that the use of ibuprofen particles having such particle size distribution and thus exhibiting a small fine powder fraction in comparison to the commonly used, commercially available grades of ibuprofen with high fine powder fractions (such as Ibuprofen 70 as provided by BASF) obviates the requirement for taste masking coatings and/or additional granulation steps with taste-masking excipients when using ibuprofen in direct oral compositions. This saves processing time and cost and reduces the overall complexity of the production process.

It was further found by the inventors that ibuprofen particles with a larger particle size than about 100 µm or even 150 µm typically produce no pronounced bitterness, or stinging or burning sensation on the tongue of the patient within the typical time it takes to swallow a direct-to-mouth powder or granule formulation, despite the fact that they are not covered with a coating which would prevent or limit wetting of the particles and/or delay their dissolution when in contact with aqueous media such as saliva.

For instance, in one embodiment of the invention, the pharmaceutical composition is substantially free of an unpleasant bitter taste and/or another unpleasant organoleptic sensation (such as a burning sensation on the mucous membranes in the mouth, tongue and throat region) for at least 20 seconds after being placed into the mouth of a human subject. In another embodiment, the pharmaceutical composition is substantially free of said unpleasant taste(s) and/or sensation(s) for at least 30 seconds. In a further embodiment, the composition exhibits only minor negative taste sensations comparable to those that are associated with ibuprofen particles which are coated and/or granulated with a taste-masking excipient, within 120 seconds, or 90 seconds, or 60 seconds, or 45 seconds, or 30 seconds, or 20 seconds upon direct oral administration. These time periods are commonly sufficient to swallow a single dose of the direct oral composition, such that hardly any noteworthy sensations of bitterness and/or burning will be experienced by the consumer.

The ibuprofen particles in the pharmaceutical composition may be provided in a granulated form. As used herein, the term 'granules' or 'granulate' merely refers to agglomerated particles prepared from a plurality of smaller, primary particles. The ibuprofen may be granulated 'in house' prior to the preparation of the pharmaceutical composition according to the invention, using any granulation technique suitable to achieve particle size distribution described herein, including dry, wet or melt granulation techniques.

Alternatively, the ibuprofen granules may be obtained commercially; for instance, in the form of various ibuprofen grades optimized for direct compression purposes, e.g. as provided by suppliers like Glatt GmbH / Pharmatrans-Sanaq AG, including grades such as ibuprofen DC 100 (consisting of 100 wt.-% ibuprofen). Such granulated ibuprofen particles have been advertised as being suitable for direct compression. However, it has never been suggested in the art that these uncoated granules as such can be used in direct-to-mouth powder or granule formulations. The skilled person would always assume that an additional coating is mandatory in order to mask the taste of the granules, or a further granulation step which combines the ibuprofen granules with additional taste-masking excipients such as MCC.

When granulating the ibuprofen in order to achieve the particle size distribution and small fine powder fraction as claimed, the use of excipients, and in particular the use of poorly water-soluble excipients, should be limited as much as possible, for instance, to ≤10 wt.-% or to ≤5 wt.-% based on the weight of the ibuprofen particles. This facilitates high drug loading of the oral direct composition as well as fast dissolution and early onset of e.g. the analgesic effect after consumption of the oral direct composition.

In one preferred embodiment, granules of 100 wt.-% ibuprofen may be used (i.e. granules substantially consisting of ibuprofen) which are prepared in a continuous spray-granulation process. For instance, this process may be a spray congealing process, such as the process described in EP1915135B1 where - in summary - the active ingredient is molten and dispersed in the form of droplets in a fluidized bed which are then shaped and/or brought to desired size by continuously guiding the melt-droplets past a stream of solidified melt particles fluidised in process gas. The term 'substantially consisting of ibuprofen' means that no further components have been added to the active ingredient ibuprofen in order to prepare the granules. Nevertheless, very small amounts of other materials may be present, such as impurities. In other words, '100 wt.-% ibuprofen' or '90 wt.-% ibuprofen' or the like, should not be misunderstood as referring to the purity grade of the ibuprofen.

Moreover, ibuprofen granules may be used of which not more than 3 wt.-% have a particle size of 100 µm or less and not more than 7 wt.-% have a particle size of 150 µm or less.

Alternatively - or in addition to granulation - the ibuprofen particles having the particle size distribution as defined herein may be obtained by sieving ibuprofen particles (originally having a larger fine particle fraction than useful for practicing the invention) prior to their incorporation into the composition, in order to remove some or all ibuprofen particles with a low sieve diameter, e.g. of less than 150 µm, or at least those in excess of 10 wt.-%; and/or to remove some or all ibuprofen particles with a particle size of less than 100 µm or less, or at least those in excess of 5 wt.-%.

This means that for the pharmaceutical composition of the invention it is in principle of little relevance whether the target particle sizes are achieved via granulation or other techniques (e.g. optimized crystallisation techniques) and/or whether the raw material already fulfils the target size requirements or needs to be classified in sieving steps first. Nonetheless, since the composition is adapted for direct oral administration, there may be further preferences, for instance with regard to upper particle size limits and/or particle shapes, which may guide the choice of specific ibuprofen and/or excipient grades as will be detailed below.

As described above, the mean particle size of the ibuprofen particles ranges from about 200 µm to about 400 µm. Optionally, it is in the range from about 230 µm to about 370 µm, or from about 260 µm to about 340 µm. In one embodiment, the median particle size (D50) falls within the same ranges. In a specific embodiment, the mean particle size falls within the range of ± 5 % of the median particle size (D50) or vice versa, or even within the range of ± 3 %. This is typically an indicator of a narrow particle size distribution (PSD) and thus helps to avoid batches containing too much fine powder and/or too many larger particles despite seemingly suitable mean values of e.g. 250 µm.

In cases where the mean particle size of the ibuprofen particles ranges from about 200 µm to about 400 µm but the PSD is rather broad, sieving steps may be required in order to obtain the target particle sizes of the invention.

In one preferred embodiment, not more than 10 wt.-% of the ibuprofen particles in the pharmaceutical composition have a sieve diameter of 500 µm or more. In other words, the D90 value of the ibuprofen particles preferably does not exceed 500 µm. In a further embodiment, not more than 5 wt.-% of the ibuprofen particles have a sieve diameter of 600 µm or more. While these particle size limitations appear to have no noteworthy effect on the taste masking of the ibuprofen, they are nonetheless preferred in that they help to reduce, or minimize, any unpleasant gritty, grainy, sandy and/or foreign body sensations of the composition when placed in the mouth and/or on the tongue.

This positive effect on mouthfeel may potentially be further enhanced if the ibuprofen particles incorporated into the composition are designed to have a substantially spheroidal shape. In one embodiment, the uncoated ibuprofen particles in at least the particle size class from 100 µm to 500 µm exhibit a substantially spheroidal or spherical shape, or in the particle size class from 150 µm to 500 µm, or in the particle size class from 150 µm to 450 µm, or in the particle size class from 200 µm to 450 µm, or in the particle size class from 200 µm to 400 µm.
As used herein, the term 'substantially spheroidal' preferably refers to particles exhibiting a mean aspect ratio (AR) more than about 0.714, or more than about 0.769, or more than about 0.833, or more than about 0.909, or more than about 0.952.
As used herein, the term 'substantially spherical' refers in particular to particles exhibiting a mean aspect ratio of more than 0.769, or more than 0.833, or more than 0.909, or more than 0.952. This distinction between spheroidal and spherical is of course chosen somewhat empirically; in practice, the transition between the two is usually smooth and without such marked boundaries.

In one embodiment, the uncoated ibuprofen particles in at least the particle size class from 100 µm to 500 µm exhibit a substantially spheroidal or spherical shape, preferably exhibiting a mean aspect ratio of more than about 0.714, or more than about 0.769, or more than about 0.833, or more than about 0.909, or more than about 0.952.

In one embodiment, the uncoated ibuprofen particles in at least the particle size class from 150 µm to 500 µm exhibit a substantially spheroidal or spherical shape, preferably exhibiting a mean aspect ratio of more than about 0.714, or more than about 0.769, or more than about 0.833, or more than about 0.909, or more than about 0.952.

In one embodiment, the uncoated ibuprofen particles in at least the particle size class from 150 µm to 450 µm exhibit a substantially spheroidal or spherical shape, preferably exhibiting a mean aspect ratio of more than about 0.714, or more than about 0.769, or more than about 0.833, or more than about 0.909, or more than about 0.952.

In one embodiment, the uncoated ibuprofen particles in at least the particle size class from 200 µm to 450 µm exhibit a substantially spheroidal or spherical shape, preferably exhibiting a mean aspect ratio of more than about 0.714, or more than about 0.769, or more than about 0.833, or more than about 0.909, or more than about 0.952.

In one embodiment, the uncoated ibuprofen particles in at least the particle size class from 200 µm to 400 µm exhibit a substantially spheroidal or spherical shape, preferably exhibiting a mean aspect ratio of more than about 0.714, or more than about 0.769, or more than about 0.833, or more than about 0.909, or more than about 0.952.

In one of the particularly preferred embodiments, the mean aspect ratio of the ibuprofen particles is from about 0.952 to about 0.714, or from about 0.909 to about 0.741; this applies to any of the above described particle size classes.

The aspect ratio (AR), also sometimes referred to as width/length ratio or breadth/length-ratio, is a shape factor used in image analysis which is defined e.g. as the ratio of the particle's width to the maximum Feret diameter (X_{c min}/X_{Fe max}). Similarly, ISO 9276-6 norm defines the aspect ratio (AR) as the ratio between the minimum and maximum Feret diameters (X_{Fe min}/X_{Fe max}). It should be noted, that the vice versa definition of the aspect ratio (i.e. 1/AR) is also commonly used, e.g. the ratio between longest diameter and shortest diameter orthogonal to it, or as the ratio of the maximum to the minimum Feret diameter (X_{Fe max}/X_{Fe min}). The above described AR-values of more than about 0.714, 0.769, 0.833, 0.909, or 0.952 would thus translate to 1/AR-values of less than about 1.4, or less than about 1.3, or less than about 1.2, or less than about 1.1, or less than about 1.05. With both approaches, a spherical particle would have the aspect ratio of 1.0.

However, as used herein, the term aspect ratio refers to the ratio X_{c min}/X_{Fe max}. When considering a multitude of particles, specifications for shape parameters such as the aspect ratio may be reported as number-based, weight-based or volume-based mean value. When referring to mean AR-values herein this is to be interpreted as the volume-based mean value; for instance expressed as the Camsizer® parameter b/l₃ (see e.g. Table 2). For reasons of simplicity, this volume-based mean value is considered to be substantially the same as a weight-based mean value for particles consisting of ibuprofen (i.e. 100 wt.-% ibuprofen; such as the above mentioned Ibuprofen DC 100 particles).

It is possible of course that the aspect ratio varies throughout different particle size classes when the particle size is analysed in more precise subdivisions (as may also be seen in e.g. Table 2). In this case, the provisions for the mean AR-values described above apply to all particle size classes; in other words, the volume based mean AR-value of all particle size classes should preferably be more than about 0.714, or more than about 0.769, or more than about 0.833, or more than about 0.909, or more than about 0.952.

Preferably, the aspect ratio is determined by automated image analysis; for instance, the Camsizer® XT device described above for the determination of the particle size distribution also calculates the aspect ratio and reports it as the volume based mean b/l₃ for all particle classes (as shown e.g. in Table 2). Therefore, in one of the preferred embodiments, the aspect ratio is determined using the Camsizer® XT device.

Particularly useful are ibuprofen particles having a particle size distribution as disclosed above (i.e. mean particle size from about 200-400 µm and ≤10 wt.-% with a sieve diameter <150 µm, and/or ≤5 wt.-% with a sieve diameter <100 µm), wherein at least the relatively large particles, such as those having a particle size of more than about 300 µm or more than about 400 µm, have a substantially spherical shape.

Such spheroidal or spherical ibuprofen particles offer the advantages of (a) pleasant and smooth mouthfeel with no or only limited grittiness perceivable upon oral consumption, as well as (b) minimum surface-to-volume ratio per particle which may support the taste-mask effect of the particles.

The spheroidal or spherical shape of the ibuprofen particles also improves the flow properties of the compositions. This is relevant for manufacture, in particular the filling of the powder composition into the primary packaging units such as sachets. At the same time, the spheroidal or spherical shape enhances the withdrawal of the composition from these units by the patient for administration.

With respect to the degree of sphericity, it is noted that typically the spheroidal or spherical shapes of the ibuprofen particles are found more often with granulated ibuprofen qualities (e.g. the above mentioned ibuprofen DC 100), while crystalline ibuprofen particles as obtained from various media are known to exhibit a hexagonal and more or less elongated, needle-like shape; with the dimensions of these crystals varying to some extent with e.g. the crystallisation medium. Therefore, granulated ibuprofen qualities exhibiting an aspect ratio of less than about 1.4 represent a preferred embodiment of the invention.

As mentioned, the ibuprofen particles are comprised in a pharmaceutical composition along with a hydrophilic polymer and at least one further pharmaceutically acceptable excipient. The pharmaceutical composition may optionally comprise from about 10 wt.-% to about 50 wt.-% ibuprofen particles; e.g. from about 15 wt.-% to about 45 wt.-%, from about 20 wt.-% to about 40 wt.-%, from about 25 wt.-% to about 35 wt.-%, such as 28 wt.-% or 33 wt.-%.

The hydrophilic polymer may be any native, synthetic or semisynthetic and physiologically inactive polymeric material which is capable of swelling, gelling, thickening and/or increasing the viscosity when in contact with aqueous media such as saliva. The hydrophilic polymer is not crosslinked. If a polymer mixture is used, the mixture may also comprise one or more constituents which are themselves not capable of swelling, gelling and/or increasing the viscosity, as long as the mixture is exhibiting these properties.

As used herein, swelling by water, or in an aqueous environment, typically means a volume increase of a solid body caused by an influx of water accompanied by hydration, i.e. wetting and absorption of moisture. Commonly, hydrophilic polymers form three-dimensional networks upon water influx and/or contact with water, entrapping the water in said networks. This is also described as gelling and results in an increase of the viscosity of water and/or the aqueous medium. The magnitude of the viscosity increase usually depends on the type and concentration of the hydrophilic polymer in the aqueous medium.

The combined processes of swelling, gelling and/or increase of the viscosity are responsible for improving the texture, or consistency, of the composition upon administration. Furthermore, vehicles with increased viscosity, such as saliva thickened by the hydrophilic polymer, may help to reduce the direct contact of ibuprofen particles with the taste buds and/or other chemo-receptor on the tongue and in the mouth in general, and thereby supplement the taste-masking effect obtained by the selection of specific particle sizes.

Examples of hydrophilic polymers suitable for the invention include, but are not limited to, water-soluble cellulose ethers such as hypromellose, hyprolose or carmellose-sodium, starches, pregelatinised starches, xanthan gums, sodium alginate, guar gum, pectin, gelatine, carrageenan and gum arabic. In one specific embodiment, the hydrophilic polymer is selected from pregelatinised starch (such as Lycatab® PGS), xanthan gum, carmellose-sodium (such as Aqualon® or Blanose® 7H4XF) or combinations thereof. In one of the preferred embodiments, the hydrophilic polymer is pregelatinised starch (such as Lycatab® PGS). It is further preferred that the preferences regarding the shape and particle size distribution of the ibuprofen particles as described above are selected in combination with the incorporation of pregelatinised starch as hydrophilic polymer.

Preferably, when employing a polymer mixture, the use of polymers which are water-insoluble or poorly water-soluble and/or which do not swell, gel, or thicken at body temperature when contacted with saliva should be limited to ≤15 wt.-%, or ≤10 wt.-%, or ≤5 wt.-% based on the total weight of the pharmaceutical composition. Since the majority of such excipients exhibiting poor aqueous solubility - including e.g. common tabletting excipients like cellulose, MCC or tricalcium phosphate - would increase the sandy, gritty feeling in the mouth and/or on the tongue upon administration of oral direct powder formulations, they should be used only in moderation and only if and to the extent that they are required. Hence, in a further preferred embodiment, the pharmaceutical composition is substantially free of such water-insoluble, non-swelling and/or non-gelling polymers.

In theory, the same concern on grittiness or a sandy mouthfeel also applies for polymers which do swell in water or aqueous media but which are poorly water-soluble or water-insoluble, such as crosslinked polymers; some of which are employed as super-disintegrants in tablet formulations, e.g. crosslinked carmellose sodium. However, since the compositions according to the invention are formulated in powder form, super-disintegrants and other crosslinked polymers are typically not required. Thus, in one embodiment, the pharmaceutical composition is substantially free of crosslinked polymers. In embodiments comprising crosslinked polymers, these would typically be used in such small amounts that their impact on mouthfeel is expected to be negligible, such as ≤ 5 wt.-% based on the weight of the pharmaceutical composition.

The weight ratio of the ibuprofen particles to the hydrophilic polymer in the compositions may optionally range from about 1 : 1 to about 20 : 1, or from about 1 : 1 to about 10 : 1; e.g. about 2 : 1, 4 : 1, 6 : 1, 8 : 1, 12 : 1, 14 : 1, or 16 : 1.

The pharmaceutical composition may comprise from about 0.5 wt.-% to about 20 wt.-% of the hydrophilic polymer, or from about 0.8 wt.-% to about 10 wt.-%, or from about 1 wt.-% to about 8 wt.-%, e.g. about 5 wt.-% or about 6 wt.-%. These values are particularly useful for pregelatinised starch, xanthan gum, and carmellose-sodium; in particular pregelatinised starch. The amount of the hydrophilic polymer may be adjusted depending on the selection of the specific polymer, keeping in mind e.g. its gel formation strength, temperature and/or ion responsiveness etc. For instance, the gel formation strength of Blanose® and xanthan gums is commonly more pronounced than that of Lycatab® PGS; hence less Blanose® and/or xanthan gum needs to be added to formulations in comparison to Lycatab® PGS.

The amount should be chosen in such a way as to provide a soft, smooth, silky, non-sticky, non-slimy and easy-to-swallow formulation when combined with the ibuprofen particles and the other excipients and ingested orally; i.e. the viscosity increase should be moderate and e.g. not lead to the compositions sticking to the tongue surface.

The pharmaceutical composition of the invention comprises at least one further pharmaceutically acceptable excipient. In one embodiment, said excipient is selected from the group of water-soluble sugars, sugar alcohols and oligosaccharides; or from the group of flavourings, cooling agents, salivation stimulating excipients, acidity regulators, lubricants and flow-regulating agents. An excipient may serve multiple purposes in the composition; it may e.g. provide a water-soluble basis as well as a sweet flavour, or regulate the acidity while at the same time stimulating salivation.

Optionally, the further excipient may also be provided in the form of granules. Optionally, the particle size distribution of the excipient granules may be similar to that of the ibuprofen particles. For instance, a sugar alcohol such as sorbitol, xylitol or maltitol may be incorporated in the form of a commercially available, granulated grade, such as Neosorb® P 300 DC (or Neosorb® XTAB 300 S), Xylisorb® 300 (or Xylisorb® XTAB) or SweetPearl® P 300 DC. These grades exhibit particle size distributions that are similar to that of the ibuprofen particles as disclosed herein.

In a specific embodiment, the sugar or sugar alcohol is selected from the group of sucrose, maltitol, mannitol, sorbitol, xylitol, and mixtures thereof, such mixtures including e.g. a blend of sorbitol with xylitol or a blend of sorbitol, xylitol and maltitol. The function of such sugar or sugar alcohol is to dissolve in the mouth and help to disperse and swallow the composition, and to provide a tasteful, sweet basis for the composition.

Suitable flavourings include but are not limited to citric acid, malic acid, succinic acid, phosphoric acid, tartaric acid and their respective salts (e.g. sodium or magnesium salts); natural, semi-synthetic and synthetic flavours, aromas (such as orange, citrus, strawberry, cherry, chocolate or mint aroma etc.); sweeteners, for example monosaccharides, disaccharides and polyhydric alcohols including arabitol, erythritol, isomalt, lactitol, maltitol, mannitol, sorbitol or xylitol; or sugar substituents, including cyclamate, saccharine, stevia, sucralose, acesulfame and/or aspartame. Further suitable flavouring compounds are freeze-dried juices and/or extracts of fruits and/or vegetables.

In one embodiment, citric acid and/or a citrate salt is employed as an acidity regulator as well as a salivation-stimulating agent, and further providing a pleasant balance between sweet and sour tastes as e.g. also found in fruits. Salivation supports the wetting of the composition and dissolution of at least the water-soluble sugar, sugar alcohol or oligosaccharide in the mouth, such that the salivated composition can be swallowed easily.

In a further embodiment, an artificial sweetener such as cyclamate, saccharine, stevia, sucralose and/or aspartame is used, alone or in combination, to supplement the sweetness of the water-soluble sugar, sugar alcohol or oligosaccharide. Optionally, the artificial sweetener, e.g. aspartame, is incorporated in granulated form.

In a yet further embodiment, one or more natural, semi-synthetic and synthetic flavours such as orange, citrus fruits, strawberry, cherry, chocolate or mint flavour is added to the composition. The flavour may be used alone or in combination; e.g. as a combination of cherry aroma and spearmint flavour. In addition to modifying the taste of the composition, some of the flavours, such as the spearmint, also provide a favourable cooling effect in the mouth. Other examples of cooling agents include other mint flavour or individual components thereof, such as menthol, limonene, or camphor.

The composition may further contain a lubricant and/or a flow-regulating agent. Stearates, in particular magnesium stearate, are examples of suitable lubricants. Fumed silica (such as Aerosil®) is an example of a flow-regulating agent, i.e. to improve the flow properties of the composition. Generally, magnesium stearate and Aerosil® should be used in small amounts, such as not more than about1 wt.-%, or even not more than about 0.5 wt.-%, in order not to affect the mouthfeel of the composition.

In one embodiment, a primary package unit comprising a single dose of the pharmaceutical composition is provided, wherein the package unit is selected from stick packs, sachets, ampoules, and vials. The presentation and oral administration in the form of stick packs, sachets, ampoules, or vials is also useful as it is possible that a relatively large amount of the composition is administered as a single dose; e.g. from about 0.5 g to about 4 g, or from about 0.75 g to about 3 g, or from about 1 g to about 2 g, e.g. 1.2 g, or 1.4 g or 1.5 g.

Optionally, the primary package unit may comprise about 50 mg to 800 mg ibuprofen. This covers the typical range of commercial ibuprofen products which has long been proven to be safe and therapeutically effective. Smaller single doses may be useful for pediatric patients in the treatment of fever and pain, while the larger doses are intended for adults.

In one specific embodiment of the invention, the pharmaceutical composition comprises spray-granulated ibuprofen particles; at least one hydrophilic polymer selected from pregelatinised starch, xanthan, carmellose-sodium and combinations thereof; and
a) a blend of water-soluble sugars, sugar alcohols and/or oligosaccharides comprising at least two components selected from sorbitol, xylitol, mannitol, sucrose and maltitol;
b) a salivation stimulating excipient selected from citric acid, monosodium citrate disodium citrate and combinations thereof;
c) one or more flavourings selected from flavours and sugar substituents; and
d) a lubricant and/or flow-regulating agent.

In a further aspect, the invention provides a process for the preparation of a pharmaceutical composition as described above, comprising the steps of:
(i) providing uncoated ibuprofen particles
   (a) which exhibit a mean particle size of ranging from about 200 µm to about 400 µm; and
   (b) of which not more than 10 wt.-% have a sieve diameter of less than 150 µm, and/or
   (c) of which not more than 5 wt.-% have a sieve diameter of less than 100 µm;
(ii) providing a hydrophilic polymer;
(iii) providing at least one further pharmaceutically acceptable excipient; and
(iv) blending all components provided in steps (i) to (iii) to form a homogenous powder composition.

Optionally, step (i) includes a granulation step and/or a sieving step to obtain the ibuprofen particles of the particle sizes described. As mentioned above, such a granulation step should not introduce larger amounts (e.g. >10 wt.-% or >5 wt.-% based on the weight of the ibuprofen particles) of excipients, and in particular poorly water-soluble excipients, into the ibuprofen particles, in order to allow for high drug loading and fast onset of effect after consumption of the oral direct composition. Granulation of the ibuprofen raw materials into particles of the sizes described herein may, for instance, involve spray congealing a melt composition comprising at least 90 wt.-%, or at least 93 wt.-%, or at least 95 wt.-%, or at least 96 wt.-%, or at least 97 wt.-%, or at least 98 wt.-%, or at least 99 wt.-% and or even 100 wt.-% ibuprofen.

The preparation of the mixture may be accomplished by any conventional blending means such as rotary-drum mixers, also known as Turbula blenders. The specific method and equipment should be selected to ensure that a) homogeneous powder compositions will be obtained in economically reasonable time frames, b) the blending does not affect the particle size of the ibuprofen particles, and c) all components are stable during the blending step.

As mentioned, the composition of the invention may be used for the treatment of pain, inflammation and/or fever due to the analgesic, anti-inflammatory and/or antipyretic properties of the drug ibuprofen.

Further potentially useful embodiments are easily derivable on the basis of the guidance provided herein-above and the following examples.

### EXAMPLES

In the following examples, the grades of the excipients that were incorporated in relatively large amounts were selected to have about the same mean particle diameters as the active ingredient (approx. 200-400 µm).

### Particle size distribution of commercially available ibuprofen grades

The particle size distribution of commercially available ibuprofen grades was determined via dynamic imaging techniques using a Camsizer® XT device (Retsch Technology GmbH, Haan, Germany), equipped with X-Jet plug-in cartridge, using the standard settings of the device.

Accordingly, the samples were air-dispersed to a density of about 0.3 % (w/V) using a dispersion pressure of about 80 kPa and a gap width of 4.0 mm. Upon passing two bright, pulsed LED light sources, the images of the dispersed particles (more specifically of their shadows) were recorded by two digital cameras, analysed for size and shape and subsequently further evaluated with regard to various parameters, including:
- the particle size, more specifically the width of the particle; i.e. the shortest chord of the measured set of maximum chords of a particle's
- the volume fraction p3 of particles within a selected particle size range x₁ to x₂ (e.g. a class from 200 µm to 250 µm) in percent (for reasons of simplicity, mass and volume fractions are considered to be substantially the same for particles consisting of ibuprofen)
- the cumulative volume fraction Q3 of all particles smaller than x i in percent (the so-called "% passing"; for reasons of simplicity, mass and volume fractions are considered to be substantially the same for particles consisting of ibuprofen)
- D10, D50 and D90, the particle size (in µm unless stated otherwise) which is passed by a mass fraction of 10 %, 50 % and 90 % of the particles, respectively
- the weighted arithmetic mean particle size (mean) and its standard deviation (SD) calculated based on the measured particle size distribution (PSD)

The ibuprofen grades analysed were:
- Conventional non-granulated ibuprofen powder from three different suppliers,
   (a) BASF, Ludwigshafen, Germany (Ibuprofen 70);
   (b) IOL Chemicals and Pharmaceuticals Limited, Ludhiana, Punjab, India;
   (c) Shasun Pharmaceuticals, Ltd., Chennai, Tamil Nadu, India.
- Granulated ibuprofen (Ibuprofen DC 100, 7 drums) by GLATT GmbH, Binzen, Germany, obtained via Pharmatrans-Sanaq AG, Basel, Switzerland.

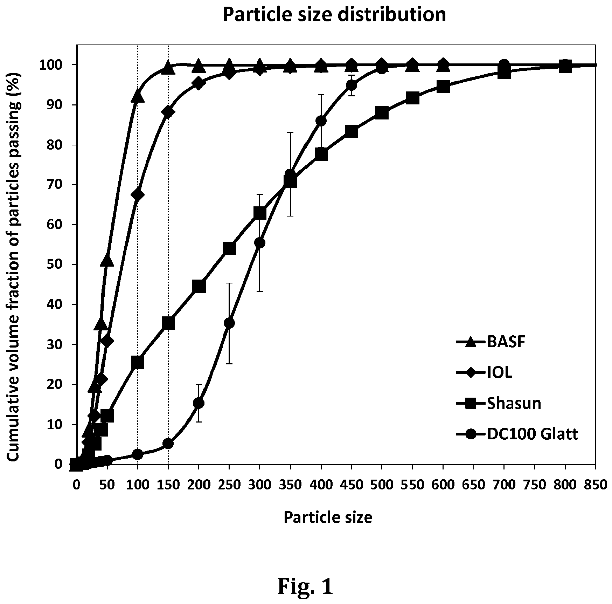

The particle size results are summarised in Table 1; the related aspect ratio values (volume based mean value b/l₃) are summarised in Table 2 (up to a cumulative fraction Q3 of 99 %). The particle size distributions are depicted in Figure 1 (cumulative volume fraction Q3 of all particles smaller than a given diameter plotted over the diameter).

As can be seen from Figure 1 and Table 1, two of the conventional ibuprofen grades (IOL, BASF) exhibit a fairly narrow particle size distribution, however, the products contain more than 85 wt.-% particles smaller than 150 µm, more than 65 wt.-% particles smaller than 100 µm and more than 30 wt.-% smaller than 50 µm. The ibuprofen obtained from Shasun contains about 35 wt.-% particles smaller than 150 µm and a very broad particle size distribution. Thus, these grades do not fulfil the particle size criteria as required according to the invention. By sieving, of course, the desired particle sizes can be obtained from these materials.

In contrast, the granulated ibuprofen has a particle size distribution according to the invention. It contains only very small fine powder fractions below 150 µm and 100 µm, respectively, and at the same time has only small amounts of particles larger than 500 µm (max. 1.6 wt.-%), and none larger than 600 µm. For all 7 measured drums, the weighted arithmetic mean value falls within the range of the D50-value ± 3 %.

As can be seen from Table 2, only the granulated ibuprofen DC 100 particles by Glatt exhibit favourable aspect ratios of more than 0.714, or more than 0.769, or more than 0.833 for particles falling in the particle size classes from about 200-400 µm. The ibuprofen particles of Shasun offer similarly favourable AR-values in the particle size classes from about 200-400 µm; however, Shasun's ibuprofen contains about 35 wt.-% particles <150 µm and exhibits a very broad particle size distribution. Thus, of the tested ibuprofen grades only the granulated ibuprofen DC 100 particles by Glatt exhibit favourable aspect ratios and particle sizes to be suited for the inventive oral direct formulation.

### Comparative example 1: Oral powder composition with coated ibuprofen

Spray-granulated ibuprofen particles (ibuprofen DC 100 by Glatt; see particle size distribution in Table 1) were coated in a hotmelt coating process with a blend of tripalmitin (Dynasan® 116) and polysorbate 65 (Tween® 65) at a ratio of 86:14 to a coating level of 30 wt.-% based on the final weight of coated particles. The coated ibuprofen was then weighed and blended with further components as listed below in a Turbula blender (Willy A. Bachofen AG, Muttenz, Switzerland) until a homogeneous powder mixture was obtained. A single dose of the mixture contained 400 mg of ibuprofen (uncoated).

| **Components** | **Composition A** |
|---|---|
| | **Content (wt.-%)** |
| Hotmelt-coated ibuprofen particles | 38.10 |
| *Ibuprofen DC 100 (Glatt)* | *26.67* |
| *Tripalmitin*/*polysorbate 65-blend 86:14* | *11.43* |
| Sorbitol | 36.57 |
| Xylitol | 19.33 |
| Citric acid (anhydrous) | 0.80 |
| Monosodium citrate (anhydrous) | 1.33 |
| Magnesium dicitrate (anhydrous) | 2.00 |
| Carmellose sodium (Na-CMC) | 1.00 |
| Aspartame | 0.07 |
| Cherry flavour | 0.10 |
| Mint flavour | 0.57 |
| Magnesium stearate | 0.13 |

The results of a comparative taste masking and mouthfeel study with 15 volunteers are described in example 4 below.

### Comparative example 2: Oral powder composition with uncoated ibuprofen and without hydrophilic polymer

The components listed below were weighed and blended in a Turbula blender (Willy A. Bachofen AG, Muttenz, Switzerland) until a homogeneous powder mixture was obtained.

| **Components** | **Composition B** |
|---|---|
| | **Content (wt.-%)** |
| Ibuprofen DC 100 | 34.39 |
| Maltitol | 17.20 |
| Sorbitol | 17.20 |
| Xylitol | 21.50 |
| Citric acid (anhydrous) | 1.29 |
| Monosodium citrate (anhydrous) | 4.30 |
| Magnesium dicitrate (anhydrous) | 2.58 |
| Aspartame | 0.09 |
| Cherry flavour | 0.09 |
| Mint flavour | 1.20 |
| Magnesium stearate | 0.17 |

When tested in volunteers, it was observed that the powder composition exhibited an unpleasant 'sandy', gritty mouthfeel; it is therefore poorly suitable for direct oral administration without water.

### Comparative example 3: Oral composition with uncoated ibuprofen, effervescent couple, and saliva-stimulating agent and without hydrophilic polymer

This composition was prepared in order to evaluate whether the formation of fine effervescent foams on the tongue in combination with the saliva-stimulating agent monosodium citrate provides taste masking. For this purpose, the components listed below were weighed and blended in a Turbula blender (Willy A. Bachofen AG, Muttenz, Switzerland) until a homogeneous powder mixture was obtained.

| **Components** | **Composition C** |
|---|---|
| | **Content (wt.-%)** |
| Ibuprofen DC 100 (Glatt) | 32.71 |
| Maltitol | 16.35 |
| Calcium carbonate (granulated, comprising 5 wt.-% maltodextrin) | 3.27 |
| Sorbitol | 16.35 |
| Xylitol | 22.08 |
| Citric acid (anhydrous) | 1.23 |
| Monosodium citrate (anhydrous) | 4.09 |
| Magnesium dicitrate (anhydrous) | 2.45 |
| Aspartame | 0.08 |
| Cherry flavour | 0.08 |
| Mint flavour | 1.14 |
| Magnesium stearate | 0.16 |

When tested in volunteers, it was observed that mouthfeel and flavour were unbalanced and not considered pleasant.

### Comparative example 4: Oral composition with uncoated ibuprofen, pregelatinised maize starch and carmellose sodium

The components listed below were weighed and blended in a Turbula blender (Willy A. Bachofen AG, Muttenz, Switzerland) until a homogeneous powder mixture was obtained. The blends contained ibuprofen with a high fine particle content (Ibuprofen 70 as obtained from BASF; see particle size distribution in Table 1) pregelatinised maize starch and carmellose sodium as the hydrophilic polymers according to the invention. A single dose of the mixture contained 400 mg of ibuprofen.

| **Components** | **Composition D** |
|---|---|
| | **Content (wt.-%)** |
| Ibuprofen 70 (BASF) | 33.33 |
| Pregelatinised maize starch | 4.17 |
| Carmellose sodium | 1.08 |
| Maltitol | 16.67 |
| Sorbitol | 16.67 |
| Xylitol | 18.92 |
| Citric acid (anhydrous) | 1.25 |
| Monosodium citrate (anhydrous) | 2.50 |
| Magnesium dicitrate (anhydrous) | 4.17 |
| Aspartame | 0.08 |
| Cherry flavour | 0.92 |
| Mint flavour | 0.08 |
| Magnesium stearate | 0.17 |

The results of a comparative taste masking and mouthfeel study with 15 volunteers are described in example 4 below.

### Example 1: Oral powder compositions with uncoated ibuprofen and carmellose sodium

The components listed below were weighed and blended in a Turbula blender (Willy A. Bachofen AG, Muttenz, Switzerland) until a homogeneous powder mixture was obtained. Carmellose sodium was used as hydrophilic polymer. Sachets were filled with approximately 1200 mg, each sachet containing a single dose of 400 mg of ibuprofen.

| **Components** | **Composition E** |
|---|---|
| | **Content (wt.-%)** |
| Ibuprofen DC 100 (Glatt) | 33.67 |
| Carmellose sodium (Na-CMC) | 2.10 |
| Maltitol | 16.84 |
| Sorbitol | 16.84 |
| Xylitol | 21.04 |
| Citric acid (anhydrous) | 1.26 |
| Monosodium citrate (anhydrous) | 4.21 |
| Magnesium dicitrate (anhydrous) | 2.53 |
| Aspartame | 0.08 |
| Cherry flavour | 0.08 |
| Mint flavour | 1.18 |
| Magnesium stearate | 0.17 |

When tested in volunteers, the composition exhibited an acceptable mouthfeel. The unpleasant bitter taste of ibuprofen was hardly noticeable.

### Example 2: Oral powder compositions with uncoated ibuprofen and xanthan gum

The components listed below were weighed and blended in a Turbula blender (Willy A. Bachofen AG, Muttenz, Switzerland) until a homogeneous powder mixture was obtained. Xanthan gum was used as the hydrophilic polymer. Sachets were filled with approximately 1200 mg, each sachet containing a single dose of 400 mg of ibuprofen.

| **Components** | **Composition F** |
|---|---|
| | **Content (wt.-%)** |
| Ibuprofen DC 100 (Glatt) | 33.67 |
| Xanthan gum | 2.10 |
| Maltitol | 16.84 |
| Sorbitol | 16.84 |
| Xylitol | 21.04 |
| Citric acid (anhydrous) | 1.26 |
| Monosodium citrate (anhydrous) | 4.21 |
| Magnesium dicitrate (anhydrous) | 2.53 |
| Aspartame | 0.08 |
| Cherry flavour | 0.08 |
| Mint flavour | 1.18 |
| Magnesium stearate | 0.17 |

When tested in volunteers, the composition exhibited an acceptable mouthfeel. The unpleasant bitter taste of ibuprofen was hardly noticeable.

### Example 3: Oral powder compositions with uncoated ibuprofen, pregelatinised starch and optionally carmellose sodium

The components listed below were weighed and blended in a Turbula blender (Willy A. Bachofen AG, Muttenz, Switzerland) until a homogeneous powder mixture was obtained. The blends contained spray-granulated ibuprofen particles (ibuprofen DC 100 by Glatt; see particle size distribution in Table 1), pregelatinised maize starch and optionally carmellose sodium as the hydrophilic polymers according to the invention. A single dose of the mixture contained 400 mg of ibuprofen.

| **Components** | **Content (wt.-%)** | |
|---|---|---|
| | **G** | **H** |
| Ibuprofen DC 100 (Glatt) | 33.33 | 28.57 |
| Pregelatinised maize starch | 4.17 | 15.50 |
| Carmellose sodium | 1.08 | --- |
| Maltitol | 16.67 | 14.29 |
| Sorbitol | 16.67 | 14.29 |
| Xylitol | 18.92 | 19.29 |
| Citric acid (anhydrous) | 1.25 | 1.07 |
| Monosodium citrate (anhydrous) | 2.50 | 3.57 |
| Magnesium dicitrate (anhydrous) | 4.17 | 2.14 |
| Aspartame | 0.08 | 0.07 |
| Cherry flavour | 0.92 | 1.00 |
| Mint flavour | 0.08 | 0.07 |
| Magnesium stearate | 0.17 | 0.14 |

When tested in volunteers, both compositions exhibited a pleasant mouthfeel and good taste masking (see example 4 below).

### Example 4: Comparative test in volunteers - Taste masking, mouthfeel, overall performance

Single dose samples of the compositions A, D and G described above each containing 400 mg ibuprofen, were evaluated by 15 volunteers at different time points with regard to e.g. taste (masking), texture and mouthfeel, and ease of swallowing. Before every test, any pre-existing intraoral taste was neutralised by drinking water. The product was not chewed.

Besides bitter taste, all other taste experiences and/or further organoleptic sensations (such as scratching, stinging, burning, smells etc.) were also noted. In questionnaires, the volunteers were asked to evaluate the overall organoleptic performance of the compositions with regard to the following parameters by marking it on a scale from 1 (full agreement; very good) to 6 (no agreement at all; very poor) and optionally explain their rating or comment further on the quality of the respective parameter:

| Parameter | Rating | Optional explanation |
|---|---|---|
| Pleasant smell? | | |
| Pleasant aroma / flavour? | | e.g. fruity, artificial? |
| Pleasant Taste? | | e.g. sweet, sour, bitter? |
| Pleasant texture / mouthfeel? | | e.g. particles, gritty, smooth? |
| Easy to swallow? | | e.g. amounts tolerable, sticky? |
| Pleasant sensation in mouth / throat? | | e.g. scratchy, stingy, burning? |
| Pleasant aftertaste? | | e.g. neutral, bitter? |

The results, or 'marks', (mean ± standard deviation) are shown below:

| Parameter | Rating | | |
|---|---|---|---|
| | Composition D | Composition A | Composition G |
| Pleasant smell? | 2.07 ± 0.92 | 2.57 ± 0.94 | 2.07 ± 0.70 |
| Pleasant aroma / flavour? | 2.47 ± 0.74 | 2.13 ± 0.83 | 2.13 ± 0.52 |
| Pleasant Taste? | 2.60 ± 0.99 | 2.03 ± 0.77 | 1.90 ± 0.54 |
| Pleasant texture / mouthfeel? | 2.33 ± 0.98 | 1.67 ± 0.72 | 1.67 ± 0.90 |
| Easy to swallow? | 2.07 ± 0.80 | 1.60 ± 0.91 | 1.67 ± 0.72 |
| Pleasant sensation in mouth / throat? | 3.90 ± 1.47 | 2.33 ± 0.90 | 2.43 ± 0.82 |
| Pleasant aftertaste? | 3.27 ± 0.90 | 2.20 ± 0.86 | 2.03 ± 0.85 |
| Pleasant overall performance? | 2.69 ± 0.65 | 2.08 ± 0.48 | 1.99 ± 0.37 |
| 1 (full agreement; very good) to 6 (no agreement at all; very poor) | | | |

The results show that, as expected, composition A (not according to the invention) with coated ibuprofen particles performs better in all categories than composition D (not according to the invention) with conventional uncoated ibuprofen particles having a high fine powder fraction.

Surprisingly, however, the inventors found that composition G according to the invention comprising the uncoated ibuprofen particles having a particle size distribution as claimed, with a very small fine powder fraction, performs equally well as composition A, and in some aspects such as smell, taste and aftertaste even better. This taste masking effect is clearly related to the particle size distribution as compositions G and D had the same composition and differed solely with regard to their ibuprofen grade.

Further, composition G was insalivated without giving a dusty, dry, slimy or sticky mouthfeel. Coarser particles were not perceived, making the compositions easy to swallow and providing an overall harmonious impression in terms of texture.

Composition G is particularly preferred by the inventors as its good organoleptic properties are achieved with a relatively low amount of excipients and because of its excellent stability.

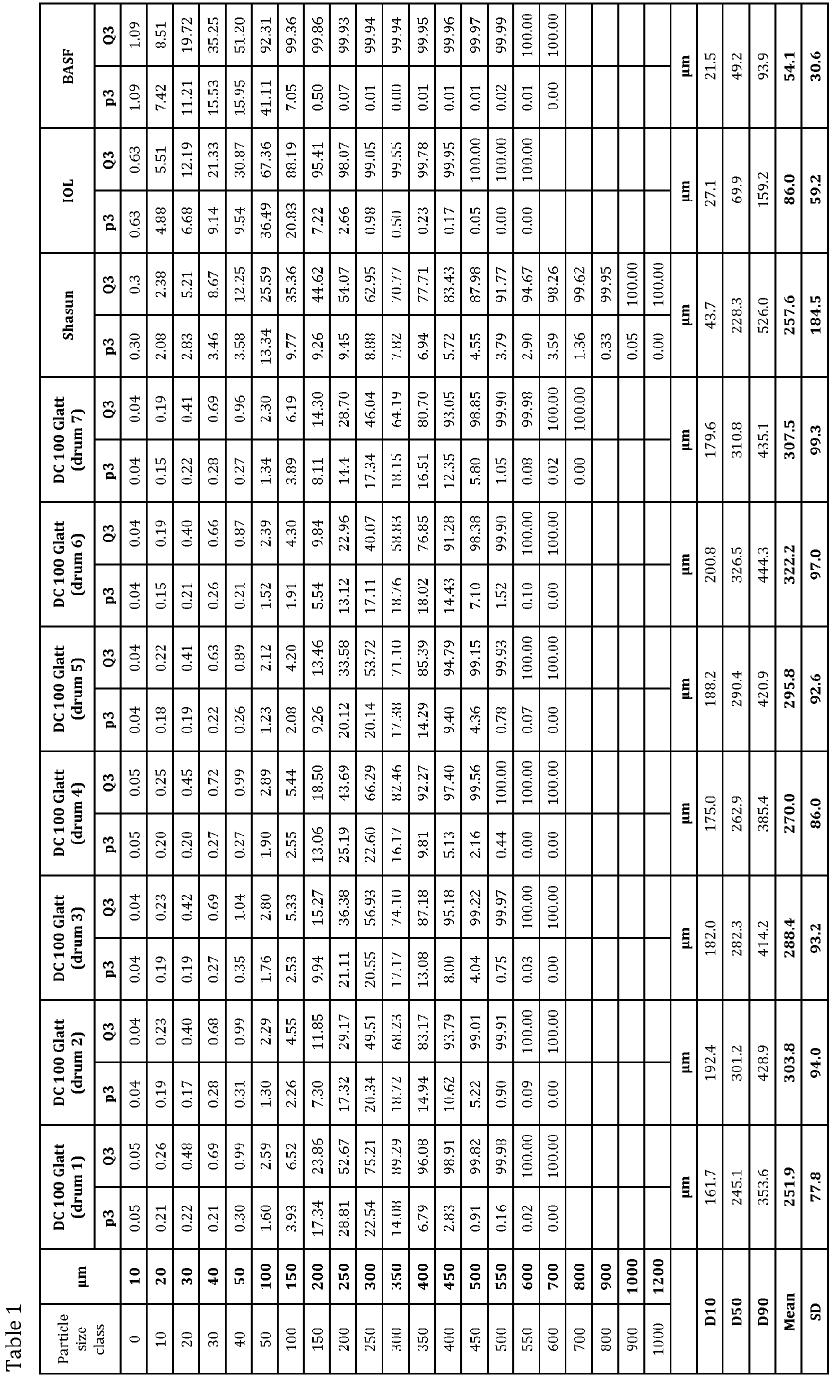

**Table 2**

| **Particle size class** | **µm** | **DC 100 Glatt (drum 1)** | **DC 100 Glatt (drum 2)** | **DC 100 Glatt (drum 3)** | **DC 100 Glatt (drum 4)** | **DC 100 Glatt (drum 5)** | **DC 100 Glatt (drum 6)** | **DC 100 Glatt (drum 7)** | **Shasun** | **IOL** | **BASF** |
|---|---|---|---|---|---|---|---|---|---|---|---|
| | | **b/13** | **b/13** | **b/13** | **b/13** | **b/13** | **b/13** | **b/13** | **b/13** | **b/13** | **b/13** |
| 0 | **10** | 0.687 | 0.706 | 0.701 | 0.696 | 0.707 | 0.688 | 0.684 | 0.654 | 0.622 | 0.647 |
| 10 | **20** | 0.671 | 0.671 | 0.689 | 0.668 | 0.671 | 0.693 | 0.684 | 0.669 | 0.639 | 0.657 |
| 20 | **30** | 0.636 | 0.641 | 0.651 | 0.615 | 0.643 | 0.650 | 0.641 | 0.663 | 0.633 | 0.643 |
| 30 | **40** | 0.658 | 0.627 | 0.656 | 0.649 | 0.702 | 0.632 | 0.643 | 0.673 | 0.646 | 0.651 |
| 40 | **50** | 0.593 | 0.625 | 0.632 | 0.610 | 0.670 | 0.644 | 0.643 | 0.668 | 0.633 | 0.634 |
| 50 | **100** | 0.690 | 0.617 | 0.640 | 0.632 | 0.650 | 0.627 | 0.627 | 0.661 | 0.609 | 0.612 |
| 100 | **150** | 0.700 | 0.668 | 0.686 | 0.696 | 0.703 | 0.654 | 0.695 | 0.695 | 0.616 | |
| 150 | **200** | 0.747 | 0.740 | 0.740 | 0.745 | 0.741 | 0.738 | 0.754 | 0.707 | 0.622 | |
| 200 | **250** | 0.778 | 0.766 | 0.773 | 0.774 | 0.771 | 0.763 | 0.764 | 0.716 | 0.625 | |
| 250 | **300** | 0.783 | 0.773 | 0.776 | 0.780 | 0.775 | 0.768 | 0.767 | 0.725 | | |
| 300 | **350** | 0.784 | 0.777 | 0.778 | 0.783 | 0.777 | 0.770 | 0.772 | 0.730 | | |
| 350 | **400** | 0.792 | 0.781 | 0.783 | 0.787 | 0.780 | 0.775 | 0.778 | 0.731 | | |
| 400 | **450** | 0.803 | 0.793 | 0.790 | 0.797 | 0.787 | 0.789 | 0.789 | 0.732 | | |
| 450 | **500** | | | | | | | 0.803 | 0.727 | | |
| 500 | **550** | | | | | | | | 0.728 | | |
| 550 | **600** | | | | | | | | 0.732 | | |
| 600 | **700** | | | | | | | | 0.757 | | |
| 700 | **800** | | | | | | | | | | |

## Claims

1. A pharmaceutical composition for direct oral administration without water formulated as a flowable powder, comprising uncoated ibuprofen particles, a hydrophilic, water-soluble, gelling polymer, which is not crosslinked, and at least one further pharmaceutically acceptable excipient, wherein
(a) the mean particle size of the ibuprofen particles ranges from 200 µm to 400 µm; and wherein
(b) not more than 10 wt.-% of the ibuprofen particles have a sieve diameter of less than 150 µm, and/or
(c) not more than 5 wt.-% of the ibuprofen particles have a sieve diameter of less than 100 µm.

2. The pharmaceutical composition according to claim 1, wherein the uncoated ibuprofen particles in at least the particle size class from 200 µm to 400 µm are spherical or spheroidal in shape, preferably exhibiting a mean aspect ratio of more than 0.714.

3. The pharmaceutical composition according to claim 1 or 2, wherein not more than 7 wt.-% of the ibuprofen particles have a sieve diameter of less than 150 µm, and/or not more than 3 wt.-% of the ibuprofen particles have a sieve diameter of less than 100 µm.

4. The pharmaceutical composition according to any preceding claim, wherein not more than 10 wt.-% of the ibuprofen particles have a sieve diameter of 500 µm or more.

5. The pharmaceutical composition according to any preceding claim, wherein the ibuprofen particles are provided in granulated form, such as in form of spray-granulated particles.

6. The pharmaceutical composition according to any preceding claim, wherein the hydrophilic, water-soluble, gelling polymer is selected from pregelatinised starch, xanthan, carmellose-sodium or combinations thereof.

7. The pharmaceutical composition according to claim any preceding claim, wherein the weight ratio of the ibuprofen particles to the hydrophilic, water-soluble, gelling polymer ranges from 1 : 1 to 20 : 1, or from 1 : 1 to 10 : 1.

8. The pharmaceutical composition according to any preceding claim, wherein the at least one further pharmaceutically acceptable excipient is selected from:
a) water-soluble sugars, sugar alcohols and oligosaccharides, and
b) flavourings, cooling agents, salivation stimulating excipients, acidity regulators, lubricants and flow-regulating agents; and
wherein the excipient may optionally be provided in the form of granules.

9. The pharmaceutical composition according to claim 8, wherein the sugar or sugar alcohol is selected from sucrose, maltitol, mannitol, sorbitol, xylitol, and mixtures thereof.

10. The pharmaceutical composition according to any preceding claim, comprising from 10 wt.-% to 50 wt.-% ibuprofen particles.

11. The pharmaceutical composition according to any preceding claim, being substantially free of an unpleasant taste and/or another unpleasant organoleptic sensation for at least 20 seconds after being placed into the mouth of a human subject, preferably for at least 30 seconds.

12. A primary package unit comprising a single dose of the pharmaceutical composition according to any preceding claim, wherein the package unit is selected from stick packs, sachets, ampoules, or vials; and/or
wherein the primary package unit comprises 50 mg to 800 mg ibuprofen.

13. The pharmaceutical composition according to any preceding claim, wherein the ibuprofen particles are spray-granulated, the hydrophilic, water-soluble, gelling polymer is selected from pregelatinised starch, xanthan, carmellose-sodium and combinations thereof; and wherein the at least one further pharmaceutically acceptable excipient includes
(a) a blend of water-soluble sugars, sugar alcohols and/or oligosaccharides comprising at least two components selected from sorbitol, xylitol, mannitol, sucrose and maltitol;
b) a salivation stimulating excipient selected from citric acid, monosodium citrate, disodium citrate and combinations thereof;
c) one or more flavourings selected from flavours and sugar substituents; and
d) a lubricant and/or flow-regulating agent.

14. The pharmaceutical composition according to any preceding claim for use in the treatment of pain, inflammation and/or fever.

15. A process for the preparation of a pharmaceutical composition according to claims 1 to 14, comprising the steps of:
(i) providing uncoated ibuprofen particles exhibiting a mean particle size ranging from 200 µm to 400 µm; wherein not more than 10 wt.-% of the ibuprofen particles have a sieve diameter of less than 150 µm and/or not more than 5 wt.-% of the ibuprofen particles have a sieve diameter of less than 100 µm;
(ii) providing a hydrophilic, water-soluble, gelling polymer which is not crosslinked;
(iii) providing at least one further pharmaceutically acceptable excipient; and
(iv) blending all components provided in steps (i) to (iii) to form a homogenous powder composition, wherein step (i) optionally includes a granulation step and/or a sieving step to obtain the ibuprofen particles.

## Patentansprüche

1. Pharmazeutische Zusammensetzung zur direkten oralen Verabreichung ohne Wasser, formuliert als ein fließfähiges Pulver, umfassend unbeschichtete Ibuprofenpartikel, ein hydrophiles, wasserlösliches, gelierendes Polymer, das nicht quervernetzt ist, und mindestens einen weiteren pharmazeutisch unbedenklichen Exzipienten, wobei
(a) die mittlere Teilchengröße der Ibuprofenpartikel im Bereich von 200 µm bis 400 µm liegt und wobei
(b) nicht mehr als 10 Gew.-% der Ibuprofenpartikel einen Siebdurchmesser von weniger als 150 µm aufweisen und/oder
(c) nicht mehr als 5 Gew.-% der Ibuprofenpartikel einen Siebdurchmesser von weniger als 100 µm aufweisen.

2. Pharmazeutische Zusammensetzung nach Anspruch 1, wobei die unbeschichteten Ibuprofenpartikel in mindestens der Teilchengrößenklasse von 200 µm bis 400 µm eine sphärische oder späroide Form aufweisen und vorzugsweise ein mittleres Aspektverhältnis von über 0,714 zeigen.

3. Pharmazeutische Zusammensetzung nach Anspruch 1 oder 2, wobei nicht mehr als 7 Gew.-% der Ibuprofenpartikel einen Siebdurchmesser von weniger als 150 µm aufweisen und/oder nicht mehr als 3 Gew.-% der Ibuprofenpartikel einen Siebdurchmesser von weniger als 100 µm aufweisen.

4. Pharmazeutische Zusammensetzung nach einem der vorhergehenden Ansprüche, wobei nicht mehr als 10 Gew.-% der Ibuprofenpartikel einen Siebdurchmesser von 500 µm oder mehr aufweisen.

5. Pharmazeutische Zusammensetzung nach einem der vorhergehenden Ansprüche, wobei die Ibuprofenpartikel in Granulatform wie in Form von sprühgranulierten Partikeln bereitgestellt werden.

6. Pharmazeutische Zusammensetzung nach einem der vorhergehenden Ansprüche, wobei das hydrophile, wasserlösliche, gelierende Polymer aus vorgelatinisierter Stärke, Xanthan, Carmellose-Natrium oder Kombinationen davon ausgewählt ist

7. Pharmazeutische Zusammensetzung nach einem der vorhergehenden Ansprüche, wobei das Gewichtsverhältnis der Ibuprofenpartikel zum hydrophilen, wasserlöslichen, gelierenden Polymer im Bereich von 1:1 bis 20:1 oder von 1:1 bis 10:1 liegt

8. Pharmazeutische Zusammensetzung nach einem der vorhergehenden Ansprüche, wobei der mindestens eine weitere pharmazeutisch unbedenkliche Exzipient ausgewählt ist aus:
a) wasserlöslichen Zuckern, Zuckeralkoholen und Oligosacchariden und
b) Geschmacksstoffen, Kühlmitteln, speichelstimulierenden Exzipienten, Säureregulatoren, Schmiermitteln und Fließregulierungsmitteln und
wobei der Exzipient gegebenenfalls in Form eines Granulats bereitgestellt werden kann.

9. Pharmazeutische Zusammensetzung nach Anspruch 8, wobei der Zucker oder Zuckeralkohol aus Saccharose, Maltit, Mannit, Sorbit, Xylit und Mischungen davon ausgewählt ist

10. Pharmazeutische Zusammensetzung nach einem der vorhergehenden Ansprüche, umfassend 10 Gew.-% bis 50 Gew.-% Ibuprofenpartikel.

11. Pharmazeutische Zusammensetzung nach einem der vorhergehenden Ansprüche, im Wesentlichen frei von einem unangenehmen Geschmack und/oder anderen unangenehmen organoleptischen Empfindungen für mindestens 20 Sekunden nach dem Platzieren im Mund eines menschlichen Subjekts, vorzugsweise für mindestens 30 Sekunden.

12. Primäre Verpackungseinheit, umfassend eine Einzeldosis der pharmazeutischen Zusammensetzung nach einem der vorhergehenden Ansprüche, wobei die Verpackungseinheit aus Stick Packs, Beuteln, Ampullen oder Vials ausgewählt ist und/oder wobei die primäre Verpackungseinheit 50 mg bis 800 mg Ibuprofen umfasst.

13. Pharmazeutische Zusammensetzung nach einem der vorhergehenden Ansprüche, wobei die Ibuprofenpartikel sprühgranuliert sind, das hydrophile, wasserlösliche, gelierende Polymer aus vorgelatinisierter Stärke, Xanthan, Carmellose-Natrium und Kombinationen davon ausgewählt ist und wobei der mindestens eine pharmazeutisch unbedenkliche Exzipient Folgendes umfasst:
(a) eine Mischung von wasserlöslichen Zuckern, Zuckeralkoholen und/oder Oligosacchariden, umfassend mindestens zwei Komponenten ausgewählt aus Sorbit, Xylit, Mannit, Saccharose und Maltit,
b) einen speichelstimulierenden Exzipienten ausgewählt aus Citronensäure, Mononatriumcitrat, Dinatriumcitrat und Kombinationen davon,
c) ein oder mehrere Geschmacksstoffe ausgewählt aus Geschmäcken und Zuckerersatzstoffen und
d) ein Schmiermittel und/oder Fließregulierungsmittel.

14. Pharmazeutische Zusammensetzung nach einem der vorhergehenden Ansprüche zur Verwendung bei der Behandlung von Schmerzen, Entzündungen und/oder Fieber.

15. Verfahren zur Herstellung einer pharmazeutischen Zusammensetzung nach einem der Ansprüche 1 bis 14, welches die folgenden Schritte umfasst:
(i) die Bereitstellung von unbeschichteten Ibuprofenpartikeln mit einer mittleren Teilchengröße im Bereich von 200 µm bis 400 µm, wobei nicht mehr als 10 Gew.-% der Ibuprofenpartikel einen Siebdurchmesser von weniger als 150 µm aufweisen und/oder nicht mehr als 5 Gew.-% der Ibuprofenpartikel einen Siebdurchmesesr von weniger als 100 µm aufweisen,
(ii) die Bereitstellung eines hydrophilen, wasserlöslichen, gelierenden Polymers, das nicht quervernetzt ist,
(iii) die Bereitstellung mindestens eines weiteren pharmazeutisch unbedenklichen Exzipienten und
(iv) das Mischen aller der in den Schritten (i) bis (iii) bereitgestellten Komponenten unter Bildung einer homogenen Pulverzusammensetzung, wobei Schritt (i) gegebenenfalls einen Granulierungsschritt und/oder einen Siebschritt zum Erhalt der Ibuprofenpartikel umfasst

## Revendications

1. Composition pharmaceutique destinée à l'administration orale directe sans eau et formulée sous la forme d'une poudre fluide, comprenant des particules d'ibuprofène non enrobées, un polymère gélifiant hydrophile et soluble dans l'eau qui n'est pas réticulé, et au moins un autre excipient pharmaceutiquement acceptable,
(a) la granulométrie moyenne des particules d'ibuprofène étant comprise entre 200 µm et 400 µm ; et
(b) pas plus de 10 % en poids des particules d'ibuprofène ayant un diamètre de tamis inférieur à 150 µm, et/ou
(c) pas plus de 5 % en poids des particules d'ibuprofène ayant un diamètre de tamis inférieur à 100 µm.

2. Composition pharmaceutique selon la revendication 1, les particules d'ibuprofène non enrobées dans au moins la classe granulométrique de 200 µm à 400 µm étant de forme sphérique ou sphéroïdale, présentant de préférence un rapport d'aspect moyen supérieur à 0,714.

3. Composition pharmaceutique selon la revendication 1 ou 2, pas plus de 7 % en poids des particules d'ibuprofène ayant un diamètre de tamis inférieur à 150 µm, et/ou pas plus de 3 % en poids des particules d'ibuprofène ayant un diamètre de tamis inférieur à 100 µm.

4. Composition pharmaceutique selon l'une quelconque des revendications précédentes, pas plus de 10 % en poids des particules d'ibuprofène ayant un diamètre de tamis de 500 µm ou plus.

5. Composition pharmaceutique selon l'une quelconque des revendications précédentes, les particules d'ibuprofène étant fournies sous forme granulée, telle que sous forme de particules granulées par pulvérisation.

6. Composition pharmaceutique selon l'une quelconque des revendications précédentes, le polymère gélifiant, hydrophile et soluble dans l'eau étant choisi parmi l'amidon pré-gélatinisé, le xanthane, la carmellose-sodium ou leurs combinaisons.

7. Composition pharmaceutique selon l'une quelconque des revendications précédentes, le rapport en poids des particules d'ibuprofène au polymère gélifiant, hydrophile et soluble dans l'eau allant de 1:1 à 20:1, ou de 1:1 à 10:1.

8. Composition pharmaceutique selon l'une quelconque des revendications précédentes, l'au moins un autre excipient pharmaceutiquement acceptable étant choisi parmi :
a) les sucres, les alcools de sucre et les oligosaccharides solubles dans l'eau, et
b) les agents aromatisants, les agents de refroidissement, les excipients stimulant la salivation, les régulateurs d'acidité, les lubrifiants et les agents régulateurs d'écoulement; et l'excipient pouvant éventuellement être fourni sous forme de granulés.

9. Composition pharmaceutique selon la revendication 8, le sucre ou l'alcool de sucre étant choisi parmi le saccharose, le maltitol, le mannitol, le sorbitol, le xylitol et leurs mélanges.

10. Composition pharmaceutique selon l'une quelconque des revendications précédentes, comprenant de 10 % en poids à 50 % en poids de particules d'ibuprofène.

11. Composition pharmaceutique selon l'une quelconque des revendications précédentes, étant sensiblement exempte de goût désagréable et/ou d'une autre sensation organoleptique désagréable pendant au moins 20 secondes après avoir été placée dans la bouche d'un sujet humain, de préférence pendant au moins 30 secondes.

12. Unité d'emballage primaire comprenant une dose unique de la composition pharmaceutique selon l'une quelconque des revendications précédentes, l'unité d'emballage étant choisie parmi les emballages en bâtonnets, les sachets, les ampoules ou les flacons ; et/ou l'unité d'emballage primaire comprenant 50 mg à 800 mg d'ibuprofène.

13. Composition pharmaceutique selon l'une quelconque des revendications précédentes, les particules d'ibuprofène étant granulées par pulvérisation, le polymère gélifiant, hydrophile et soluble dans l'eau étant choisi parmi l'amidon pré-gélatinisé, le xanthane, la carmellose-sodium et leurs combinaisons ; et l'au moins un autre excipient pharmaceutiquement acceptable comprenant
a) un mélange de sucres, d'alcools de sucre et/ou d'oligosaccharides solubles dans l'eau comprenant au moins deux composants choisis parmi le sorbitol, le xylitol, le mannitol, le saccharose et le maltitol ;
b) un excipient stimulant la salivation choisi parmi l'acide citrique, le citrate monosodique, le citrate disodique et leurs combinaisons ;
c) un ou plusieurs agents aromatisants choisis parmi les arômes et les substituants de sucre ; et
d) un lubrifiant et/ou un agent de régulation du débit.

14. Composition pharmaceutique selon l'une quelconque des revendications précédentes destinée à être utilisée dans le traitement de la douleur, de l'inflammation et/ou de la fièvre.

15. Procédé de préparation d'une composition pharmaceutique selon les revendications 1 à 14, le procédé comprenant les étapes suivantes :
(i) fournir des particules d'ibuprofène non enrobées présentant une granulométrie moyenne de 200 µm à 400 um ; pas plus de 10 % en poids des particules d'ibuprofène ayant un diamètre de tamis inférieur à 150 µm, et/ou pas plus de 5 % en poids des particules d'ibuprofène ayant un diamètre de tamis inférieur à 100 µm.
(ii) fournir un polymère gélifiant, hydrophile et soluble dans l'eau qui n'est pas réticulé ;
(iii) fournir au moins un autre excipient pharmaceutiquement acceptable ; et
(iv) mélanger tous les composants fournis dans les étapes (i) à (iii) pour former une composition de poudre homogène, l'étape (i) incluant facultativement une étape de granulation et/ou une étape de tamisage pour obtenir les particules d'ibuprofène.
